Europäisches Patentamt

European Patent Office

Office européen des brevets

(19)

(11) Publication number: **0 152 023**
**A2**

(12) **EUROPEAN PATENT APPLICATION**

(21) Application number: **85101006.6**

(22) Date of filing: **31.01.85**

(51) Int. Cl.⁴: **A 61 L 2/12**
**//A61F13/00**

(30) Priority: **09.02.84 US 578719**

(43) Date of publication of application:
**21.08.85 Bulletin 85/34**

(84) Designated Contracting States:
**BE DE FR GB LU NL**

(71) Applicant: **KIMBERLY-CLARK CORPORATION**
**401 North Lake Street**
**Neenah Wisconsin 54956(US)**

(72) Inventor: **Schiffmann, Robert Frank**
**149 West 88th Street**
**New York New York(US)**

(72) Inventor: **Robinson, James Edgar**
**227 Crescent Drive**
**Neenah Wisconsin(US)**

(74) Representative: **Patentanwälte Grünecker, Dr.**
**Kinkeldey, Dr. Stockmair, Dr. Schumann, Jakob, Dr.**
**Bezold, Meister, Hilgers, Dr. Meyer-Plath**
**Maximilianstrasse 58**
**D-8000 München 22(DE)**

(54) Sanitizing method for absorbent articles.

(57) A method is provided for sanitizing cellulosic absorbent articles such as tampons, diapers and the like by heating the packaged article to a temperature of at least 90°C for a period of at least 2 minutes while the ambient temperature is maintained at 100°C.

# SANITIZING METHOD FOR ABSORBENT ARTICLES 0152023

## FIELD OF THE INVENTION

This invention relates to a method for sanitizing absorbent articles and particularly cellulosic derived absorbent articles such as tampons and diapers and the like.

## BACKGROUND OF THE INVENTION

There are currently a variety of commercially available products which are designed to absorb a variety of biological fluids such as a urinary secretions, menstrual exudate and fluid drainage from surgical wounds. For purposes of this application, these products will generally be referred to as secretafacients. These products are generally bulky, are good insulators, are designed to absorb varying amounts of fluid, and are generally cellulosic in nature. The term cellulosic, as herein used, refers also to materials such as rayon and the like which are derived from cellulose as well as wood pulp fibers which are cellulosic in origin.

These products which are used to absorb secretions from a body orifice or a wound, are generally treated carefully during manufacturing to eliminate, or at least minimize, microbiological contamination. Regardless of how carefully these products are treated, occasionally microbiological contamination may be present. Tampons, particularly due to their use internally, should be free of harmful microbiological contamination. Treatments for minimizing or eliminating contamination are known, e. g. sterilizing with ethylene oxide.

Tampons differ from the other secretafacient materials in yet another way. The traditional tampon which is encased in an inserter has been compressed to form a comparatively dense cellulosic mass. The interior portions of the tampon, therefore, become difficult to treat with any conventional sanitizing medium. Due to its relatively dense character, the application of conventional heat sources may deteriorate the outer surface of the tampon prior to the introduction of a sufficiently high temperature level at the interior. This limits the effectiveness of conventional heat processing as a microbiologically inhibiting treatment. Also, due to the dense compressed nature of the tampon, introduction of a sanitizing or sterilizing gas such as ethylene oxide is difficult. Further, it has been noted that ethylene oxide has carcinogenic activity and its contact with the human body should be avoided.

Tampons and similar items should ideally be sterilized after packaging to prevent recontamination during manufacturing. However, when several densified tampons are packaged together the insulating effect of these tampons in relation to each other is substantial and heating and sterilizing the individual tampon interiors and surface portions has become even more difficult.

Further, since boxes of tampons are wrapped with an inexpensive, but heat sensitive plastic barrier film, to prevent recontamination, heating of the finished package becomes even more limited.

Ionizing radiation has been tried but degrades the tampon pledget. Utilization of convection heating alone has proven to be impossible due to the extremely long times needed and the damage resulting to the barrier film.

## SUMMARY OF THE INVENTION

According to this invention, a method for sanitizing bulky cellulosic secretafacient devices is provided which eliminates the possibility of subsequent contamination after treatment and also provides a simple in-line process which can be coupled to most manufacturing operations. The method of this invention is concerned with the application of heat sufficient to raise and maintain both the interior and the exterior temperature of the bulky cellulosic secretafacient device after the secretafacient device has been packaged.

## DETAILED DESCRIPTION OF THE INVENTION

According to this invention a secretafacient device such as a tampon is conventionally manufactured and packaged. These products can be individually packaged or, more commonly, packed in the traditional merchandising type of package which contains anywhere from 12 to 30 separate articles in either a cardboard box or polyethylene bag.

The packages are then conveyed through a heating chamber in which a means for heating the interior of the secretafacient to a desired temperature is coupled with a means for maintaining an elevated temperature at the inner and outer portion of the secretafacient. The heating process which is the subject of this invention is designed to relatively rapidly obtain and maintain substantially equal temperatures on the outside surface and the interior portion of the device.

It has been found that rapidly heating the secretafacient at the interior portion without destroying the desired absorbent or other physical properties of the secretafacient product can be accomplished only by utilizing microwave energy. High level microwave treatments rapidly heat the interior portions of a secretafacient, such as a tampon, much faster than the exterior surface. Microwave energy is relatively effective and a rapid way of obtaining the desired temperature in the interior portion of these products.

Subsequently, in order to maintain the interior temperature of the secretafacient, the secretafacient is subjected to a further reduced energy level microwave heat treatment. This heat treatment can be, for example, low level or pulsed microwave. By so doing, the dissipation of heat from the interior is controlled and minimized. Temperatures at the interior and exterior are balanced and equalized thereby providing suitable simultaneous microbiologically destructive treatments throughout the article. This second reduced energy level microwave treatment has proven to be absolutely essential for controlling microbial growth without resultant product 0152023 destruction.

It has been found that significant decrease in the amount of vegetatively growing pathogenic bacteria occur when the internal temperature is maintained at least at 90°C. and ambient temperatures with hold times of 2 minutes at 100°C. are utilizied. As will be illustrated from the example below, complete commercial sterilization can be obtained, at least for some specific pathogens, when internal and external temperatures are properly elevated.

## EXAMPLE 1

KOTEX® Security Tampons were sterilized, subjected to an inoculation consisting respectively of $10^3$ and $10^6$ organisms of staphyloccus aureus in .05 milliliters of water with the designation C to show center innoculation and S to show surface innoculation. These thoroughly innoculated tampons were packaged in a conventional KOTEX Security box containing 30 tampons and subjected to a variety of heat treatments and microwave inputs. All of the tampons were maintained in a microwave oven providing internal tampon temperatures of 90°C. The tampons were subjected to microwave energy in a Hirst Micro-Aire Oven S/N C-104 having a power output of 0 to 2 KW (adjustable), 1 phase, 60 Hz, at 210 volts and a 3 KW hot air supply. This unit is made by Hirst Microwave Industries, Industrial Estate, Spinney Lane, Aylesham NR. Canterbury, Kent, England. These samples were treated under the conditions indicated in the table below:

TABLE I                0152023

| Sample Ident. | Tampon Center/ Surface Temp. °C. | Oven Temp. °C. | Come-Up Time Min. | Hold Time Min. | Added Micro- wave ? | % Survivors | | | |
|---|---|---|---|---|---|---|---|---|---|
| | | | | | | $10^3$ | | $10^6$ | |
| | | | | | | S* | C** | S* | C** |
| M4-60-1 | 61/61 | AMB. | 1 | 0 | No | 100 | 100 | 100 | 100 |
| M4-60-2 | 61/65 | AMB. | 1 | 1 | Yes | 100 | 100 | 100 | 100 |
| M4-60-3 | 60/62 | AMB. | 1 | 2 | Yes | 100 | 100 | 100 | 100 |
| M4-60-4 | 60/56 | 100 | 1 | 0 | No | 100 | 100 | 100 | 100 |
| M4-60-5 | 57/61 | 100 | 1 | 1 | No | 100 | 100 | 100 | 100 |
| M4-60-6 | 57/56 | 100 | 1 | 2 | No | 100 | 100 | 100 | 100 |
| M4-60-7 | 65/? | 100 | 1 | 1 | Yes | 100 | 100 | 100 | 100 |
| M4-60-8 | 61/58 | 100 | 1 | 2 | Yes | 100 | 100 | 100 | 100 |
| M4-60-9 | 62/54 | AMB. | 2 | 0 | No | 100 | 100 | 100 | 100 |
| M4-60-10 | 60/61 | AMB. | 2 | 1 | Yes | 100 | 100 | 100 | 100 |
| M4-60-11 | 60/60 | AMB. | 2 | 2 | Yes | 0 | 100 | 100 | 100 |
| M4-60-12 | 60/54 | 100 | 2 | 0 | No | 100 | 100 | 100 | 100 |
| M4-60-13 | 57/57 | 100 | 2 | 1 | No | 100 | 100 | 100 | 100 |
| M4-60-14 | 57/61 | 100 | 2 | 2 | No | 100 | 100 | 100 | 100 |
| M4-60-15 | 63/64 | 100 | 2 | 1 | Yes | 100 | 100 | 100 | 100 |
| M4-60-16 | 60/50 | 100 | 2 | 2 | Yes | 100 | 100 | 100 | 100 |
| M4-75-1 | 76/67 | AMB. | 1 | 0 | No | 100 | 100 | 100 | 100 |
| M4-75-2 | 77/76 | AMB. | 1 | 1 | Yes | 0 | 0 | 100 | 100 |
| M4-75-3 | 75/73 | AMB. | 1 | 2 | Yes | 0 | 100 | 100 | 100 |
| M4-75-4 | 76/75 | 100 | 1 | 0 | No | 100 | 0 | 100 | 100 |
| M4-75-5 | 69/66 | 100 | 1 | 1 | No | 100 | 100 | 100 | 100 |
| M4-75-6 | 69/66 | 100 | 1 | 2 | No | 100 | 100 | 100 | 100 |
| M4-75-7 | 76/72 | 100 | 1 | 1 | Yes | 0 | 0 | 100 | 0 |
| M4-75-8 | 76/69 | 100 | 1 | 2 | Yes | 0 | 0 | 0 | 100 |
| M4-75-9 | 75/65 | AMB. | 2 | 0 | No | 0 | 100 | 100 | 100 |
| M4-75-10 | 76/71 | AMB. | 2 | 1 | Yes | 100 | 0 | 100 | 100 |
| M4-75-11 | 76/78 | AMB. | 2 | 2 | Yes | 100 | 100 | 100 | 100 |
| M4-75-12 | 76/68 | 100 | 2 | 0 | No | 100 | 100 | 100 | 100 |
| M4-75-13 | 72/68 | 100 | 2 | 1 | No | 100 | 100 | 100 | 100 |
| M4-75-14 | 70/66 | 100 | 2 | 2 | No | 100 | 100 | 100 | 100 |
| M4-75-15 | 76/71 | 100 | 2 | 1 | Yes | 0 | 0 | 100 | 100 |
| M4-75-16 | 74/68 | 100 | 2 | 2 | Yes | 0 | 0 | 100 | 100 |
| M4-90-1 | 91/84 | AMB. | 1 | 0 | No | 0 | 0 | 0 | 100 |
| M4-90-2 | 90/81 | AMB. | 1 | 1 | Yes | 100 | 0 | 100 | 100 |
| M4-90-3 | 90/79 | AMB. | 1 | 2 | Yes | 0 | 0 | 100 | 0 |
| M4-90-4 | 91/81 | 100 | 1 | 0 | No | 0 | 0 | 0 | 0 |
| M4-90-5 | 92/81 | 100 | 1 | 1 | No | 0 | 0 | 0 | 0 |
| M4-90-6 | 88/78 | 100 | 1 | 2 | No | 0 | 0 | 100 | 0 |
| M4-90-7 | 92/82 | 100 | 1 | 1 | Yes | 0 | 0 | 0 | 0 |
| M4-90-8 | 90/83 | 100 | 1 | 2 | Yes | 0 | 0 | 0 | 0 |
| M4-90-9 | 91/78 | AMB. | 2 | 0 | No | 0 | 0 | 100 | 100 |
| M4-90-10 | 90/79 | AMB. | 2 | 1 | Yes | 0 | 0 | 0 | 100 |
| M4-90-11 | 90/82 | AMB. | 2 | 2 | Yes | 0 | 0 | 0 | 0 |
| M4-90-12 | 90/84 | 100 | 2 | 0 | No | 0 | 0 | 0 | 0 |
| M4-90-13 | 85/78 | 100 | 2 | 1 | No | 0 | 0 | 0 | 0 |
| M4-90-14 | 81/78 | 100 | 2 | 2 | No | 100 | 0 | 0 | 100 |
| M4-90-15 | 91/84 | 100 | 2 | 1 | Yes | 0 | 0 | 0 | 0 |
| M4-90-16 | 91/80 | 100 | 2 | 2 | Yes | 0 | 0 | 0 | 0 |

S*= Surface

C**= Center

Where added microwave is shown on the table above the time for the added microwave exposure at reduced power is equal to the hold time. Hold time is the time after the indicated internal temperature of the tampon is obtained in which the product is maintained in the oven. Where the oven shows a temperature other than ambient, this means that the tampon package has been subjected to external convection heating for a period equal to the hold time and the come-up time, come-up time being the time used to bring the center of the tampon to the temperature indicated in the table.

Added microwave is always lower energy level (in this case pulsed) used to maintain center temperature.

Tampon temperature values are measured after hold time although the inner probe indicated in each test that the center temperature obtained at the end of come-up time was identical.

The data indicates that reduced energy microwave hold time of 2 minutes is sufficient to prevent internal microbial growth even where there is only ambient temperature maintained. It is also clear that surface growth can be controlled or eliminated by this lower energy second stage heating, although the addition of convection heating as a safety factor is preferred.

It should be noted that growth was measured after a 7-day incubation period.

WHAT IS CLAIMED IS:

1. Method of microbiological control of growth in a package of individual cellulosic units comprising:

    a) subjecting the package to a first microwave energy level to obtain a desired elevated temperature at the center of said individual units;

    b) subjecting said package to a second microwave energy level to maintain said temperature with said second energy level being lower than said first energy level; and

    c) providing heating conditions for obtaining and maintaining temperature levels throughout said units equal to those maintained at the center.

2. The method according to Claim 1 wherein the temperature at the center of the individual units is 90°C. and said temperature is maintained for at least 2 minutes.

3. The method according to Claim 1 wherein temperature is maintained by pulsed microwave energy.